# EUROPEAN PATENT APPLICATION

(11) **EP 4 074 347 A1**
(43) Date of publication of application: **19.10.2022**
(21) Application number: 20908578.6
(22) Date of filing: 18.08.2020
(51) Int. Cl.: A61L 2/20, A61L 2/07, A61L 9/03, B05B 9/00

(54) **HYDROGEN PEROXIDE VAPOR GENERATION DEVICE, SPACE STERILIZATION APPARATUS INCLUDING SAME, AND SPACE STERILIZATION METHOD**

(30) Priority: 02.01.2020 KR 20200000254; 20.05.2020 KR 20200060600; 26.06.2020 KR 20200078344
(71) Applicant: Huons Meditech Co., Ltd., Jungwon-gu Seongnam-si, Gyeonggi-do 13201 (KR)
(72) Inventor: LEE, Sang Man, Anyang-si Gyeonggi-do 14106 (KR); KIM, Kang Hyun, Incheon 21306 (KR); KANG, In Han, Gwangju-si Gyeonggi-do 12765 (KR)
(74) Representative: Manitz Finsterwald Patent- und Rechtsanwaltspartnerschaft mbB
(86) International application number: PCT/KR2020/010970
(87) International publication number: WO 2021/137381

(57) **Abstract**

The present invention relates to a hydrogen peroxide vapor generation device, a space sterilization apparatus including same, and a space sterilization method, including: an evaporation chamber which has a space formed therein, and includes a nozzle unit and a discharge port which are formed in an upper portion thereof; an evaporation unit which is formed in a lower inner portion of the evaporation chamber and equipped with a plurality of heating units and a hydrogen peroxide transfer pipe which are arranged in stack; and an air supply unit which is formed in a lower portion of the evaporation chamber and through which outside air is suctioned, discharged, and fed to the evaporation unit.

## Description

### Technical Field

The present invention relates to a hydrogen peroxide vapor generation device capable of sterilizing an object to be sterilized with a hydrogen peroxide which is a kind of chemical sterilizing agent, a space sterilization apparatus including the same, and a space sterilization method.

### Background Art

In general, sterilization and disinfection of medical instruments, and the like are usually performed by using high pressure steam sterilization method using saturated steam under high pressure, gas sterilization method using chemical substances that do not require high temperature, high humidity and high pressure and do not damage instruments or materials, or the like.

Since it was confirmed that the hydrogen peroxide can help the immune system and various body repair systems resolve infections or damage by not only reducing microbial levels but also cleaning wounds, hydrogen peroxide has been used as a powerful oxidizing agent. Initially, this solution, which was used for sterilization and disinfection purposes, started to be used by way of direct application to the skin. However, this method caused direct damage to the body while oxidizing the target microorganisms and the like. Therefore, the solution was used in forms that are determined according to the purposes and method of use while controlling its concentration.

Hydrogen peroxide in aerosol or gaseous state is used to purify air. Currently, hydrogen peroxide vapor is widely used in places that require sterilization, such as hospitals, research institutes, drug and food manufacturing processes, and the like.

Hydrogen peroxide in gaseous form has far more advantages as an antimicrobial agent than in liquid state. First of all, even a low concentration of hydrogen peroxide is very effective according to the exposure time, and it is effective against various microorganisms and bacteria. Many studies have shown that hydrogen peroxide is effective against mycobacteria, viruses, fungi, spores, protozoa, and the like. However, there are complex multi-variable issues to solve, such as spatial characteristics of the place requiring sterilization, the level of microorganisms to be achieved, the need for preventing condensation of the hydrogen peroxide gas injected into the space, various factors such as the possibility that peroxide gas can damage the facilities inside the space, and the like, and various methods have been proposed to solve the above.

As an example, a method is known, which causes phase change of a liquid peroxide into a gaseous state by making a hydrogen peroxide solution meet a high-temperature heat source inside an apparatus to cause forced evaporation, and transfers the evaporated hydrogen peroxide with a proper carrier gas for use of the same. In this case, the hydrogen peroxide mixture solution at atmospheric pressure and room temperature is brought into direct contact with a hot energized metal plate for evaporation to be instantaneously evaporated.

Meanwhile, in recent years, a method of evaporating the hydrogen peroxide mixture solution by increasing the temperature of the carrier gas itself is known.

The conventional method of hydrogen peroxide evaporation uses simple spraying with hot air and atomizer nozzle, or spraying the vapor instantaneously evaporated from drops falling onto hot plate with hot air, but there is a problem in that it is difficult to reach the concentration for sterilization of a large space because the amount of hydrogen peroxide that can be evaporated with these methods is small, and large amount of time and many equipment are required to sterilize a large space.

### Detailed Description of Invention

### Technical Problem

The present invention has been proposed to solve the problems of the related art, and an object of the present invention is to provide a hydrogen peroxide vapor generation device having a chamber, in which air to form an air cushion is supplied, and a mixed solution of hydrogen peroxide as an antibacterial fluid to be evaporated or water is supplied to the chamber with specific flow characteristics through the nozzle, while simplifying the complex structures inside the evaporation chamber to have a form in which the above two fluids can properly exchange energy, and reducing the necessary volume of the evaporation chamber compared to the existing method, to thus efficiently generate hydrogen peroxide vapor.

In addition, according to the present invention, it is possible to evaporate a large amount of hydrogen peroxide in a short time, so that it is possible to shorten the time and maximize the efficiency of the equipment.

In addition, the present invention includes an evaporation pipe in the shape of a coil for evaporation, an arrangement structure with a fin heater, a hot air plate and a nozzle.

### Technical Solution

The object of the present invention described above may be achieved by a hydrogen peroxide vapor generation device including: an evaporation chamber which has a space formed therein, and includes a nozzle unit and a discharge port which are formed in an upper portion thereof; an evaporation unit formed in a lower inner portion of the evaporation chamber and equipped with a plurality of heating units arranged in a stack, a heating body formed on an outer side of the evaporation chamber and formed with a heating wire, and a hydrogen peroxide transfer pipe which is mounted inside the heating body and through which hydrogen peroxide is transferred; and an air supply unit which is formed in a lower portion of the evaporation chamber and through which outside air is suctioned, discharged, and fed to the hydrogen peroxide vapor generation device.

The plurality of heating units may include a first heating unit and a second heating unit, and the hydrogen peroxide transfer pipe may be arranged between the second heating unit and the first heating unit in stack. Each of the plurality of heating units includes a tube through which a high-temperature fluid flows, and a plurality of heat dissipation fins formed on an outer surface of the tube.

The air supply unit includes a fan housing having an exhaust port formed on one side and an intake port formed on the other side, in which the exhaust port is coupled in communication with a lower opening of the evaporation chamber, and a fan rotatably mounted inside the fan housing.

The evaporation chamber includes a hot air plate formed at a position corresponding to an upper portion of the evaporation unit.

The nozzle unit includes a nozzle body having both ends open, and including a passage formed inside, a liquid supply hole formed on one side, through which a hydrogen peroxide solution is introduced, and an air distribution unit distributing and discharging air in the passage formed inside; a nozzle holder coupled to the nozzle body and including a nozzle fixing part to which the nozzle is fitted and coupled; and a liquid supply nipple coupled to the liquid supply hole and coupled to a hose connected to an external hydrogen peroxide solution storage container through the member.

The nozzle body includes a liquid discharge path formed therein in communication with the liquid supply hole, and a nozzle formed on and protruding from a lower end of the nozzle body, wherein the nozzle has a discharge hole formed at an end thereof.

The object of the present invention described above can be achieved by a space sterilization apparatus including: an enclosure including an accommodating space formed therein, and an openable cover; a discharge hole formed in an upper portion of the enclosure; a hydrogen peroxide storage tank for supplying hydrogen peroxide to the hydrogen peroxide vapor generation device; and a compressor for pressurizing and discharging hydrogen peroxide.

The object of the present invention described above may be achieved by a space sterilization apparatus including: an enclosure including an accommodating space formed therein, and an openable cover; a discharge hole formed in an upper portion of the enclosure; a hydrogen peroxide vapor generation device formed inside the enclosure; a hydrogen peroxide storage tank for supplying hydrogen peroxide to the hydrogen peroxide vapor generation device; and a compressor for pressurizing and discharging hydrogen peroxide, in which the hydrogen peroxide vapor generation device includes: an evaporation chamber which has a space formed therein, and includes a nozzle unit and a discharge port which are formed in an upper portion thereof; an evaporation unit formed in a lower inner portion of the evaporation chamber and equipped with a plurality of heating units arranged in a stack, a heating body formed on an outer side of the evaporation chamber and formed with a heating wire, and a hydrogen peroxide transfer pipe which is mounted inside the heating body and through which hydrogen peroxide is transferred; and an air supply unit which is formed in a lower portion of the evaporation chamber and through which outside air is suctioned, discharged, and fed to the heating units.

In addition, the object of the present invention can be achieved by a space sterilization apparatus including: an enclosure including an accommodating space formed therein, and an openable cover; a discharge hole formed in an upper portion of the enclosure; a perforated portion formed in the enclosure and formed with air permeability, and a filter unit mounted in communication with the perforated portion; a hydrogen peroxide vapor generation device formed inside the enclosure; a hydrogen peroxide storage tank for supplying hydrogen peroxide to the hydrogen peroxide vapor generation device; and a compressor for pressurizing and discharging hydrogen peroxide, in which the hydrogen peroxide vapor generation device includes: an evaporation chamber which has a space formed therein, and includes a nozzle unit and a discharge port which are formed in an upper portion thereof; an evaporation unit formed in a lower inner portion of the evaporation chamber and including a plurality of heating units arranged in a stack, and a hydrogen peroxide transfer pipe which is arranged between the plurality of heating units in stack and through which hydrogen peroxide is transferred; and an air supply unit which is formed in a lower portion of the evaporation chamber and through which outside air is suctioned, discharged, and fed to the heating units.

The air supply unit includes a fan housing having an exhaust port formed on one side and an intake port formed on the other side, in which the exhaust port is coupled in communication with a lower opening of the evaporation chamber, and a fan rotatably mounted inside the fan housing.

The object of the present invention described above may be achieved by a sterilization method using a space sterilization apparatus having a hydrogen peroxide vapor generation device, which starts operating as the start button is operated and sequentially performs the preheating step, the spraying step, the sterilizing step, the cooling and terminating step.

### Advantageous Effects

According to the present invention, it is possible to evaporate a large amount of hydrogen peroxide within a short time, and it is possible to shorten the time and maximize efficiency of the equipment.

According to the present invention, hydrogen peroxide is vaporized through high-temperature heating, supplied to the nozzle, and split into small particles at the nozzle, and the air supplied through the air supply unit is heated to a high temperature, meets with hydrogen peroxide split into small particles inside the evaporation chamber, is formed into smaller particles, and is discharged to the outside, such that there is an advantage of effective space sterilization.

In addition, by forming air cushions of constant shape inside the chamber rather than roughly mixing the hot air and hydrogen peroxide drops irregularly inside the chamber, control is easy, and there is a positive effect on the durability and efficiency of the inside of the chamber.

### Brief Description of Drawings

FIG. 1 is a perspective view illustrating a space sterilization apparatus according to the present invention.
FIG. 2 is a front view illustrating a space sterilization apparatus according to the present invention.
FIG. 3 is a perspective view illustrating a 'hydrogen peroxide vapor generation device' in the space sterilization apparatus according to the present invention.
FIG. 4 is an exploded perspective view illustrating the 'hydrogen peroxide vapor generation device' in the space sterilization apparatus according to the present invention.
FIG. 5 is a cross-sectional perspective view of the 'hydrogen peroxide vapor generation device' in the space sterilization apparatus according to the present invention.
FIG. 6 is an exploded perspective view of a part of the 'hydrogen peroxide vapor generation device' in the space sterilization apparatus according to the present invention.
FIG. 7 is a perspective view of a 'hydrogen peroxide vapor generation device' according to another embodiment.
FIG. 8 is an exploded perspective view of a 'nozzle unit' in the space sterilization apparatus according to the present invention.
FIG. 9 is a cross-sectional perspective view of the 'nozzle unit' in the space sterilization apparatus according to the present invention.
FIG. 10 is a bottom perspective view of the 'nozzle unit' in the space sterilization apparatus according to the present invention.

### Best Mode For Embodying Invention

According to the present invention, a space sterilization apparatus 100 includes an enclosure 110 including an accommodating space formed therein, and an openable cover; a discharge hole 122 formed in an upper portion of the enclosure 110; a storage tank 140 mounted inside the enclosure 110 and storing hydrogen peroxide and supplying the hydrogen peroxide to a hydrogen peroxide vapor generation device; a compressor 150 mounted in a lower inner portion of the enclosure 110 and supplying compressed air to a nozzle; and the hydrogen peroxide vapor generation device (A) that evaporates the hydrogen peroxide supplied from the storage tank 140.

### Mode for Embodying Invention

Hereinafter, preferred embodiments of the present disclosure will be described in detail with reference to the accompanying drawings.

The terms to be described below are defined in consideration of functions in the present disclosure, and it is noted that they should be interpreted as a concept consistent with the technical idea of the present disclosure and a meaning commonly or generally recognized in the art.

Further, in the following description of the present disclosure, a detailed description of known functions and configurations incorporated herein will be omitted when it may make the subject matter of the present disclosure rather unclear.

Here, the accompanying drawings are partially exaggerated or simplified for convenience and clarity of explanation and understanding of the configuration and operation of the technology, and each component does not exactly match the actual size.

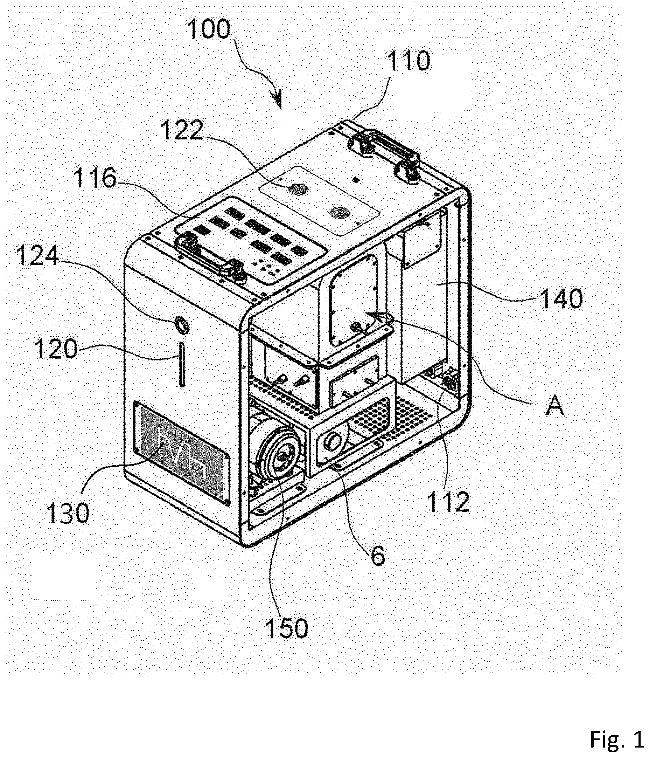

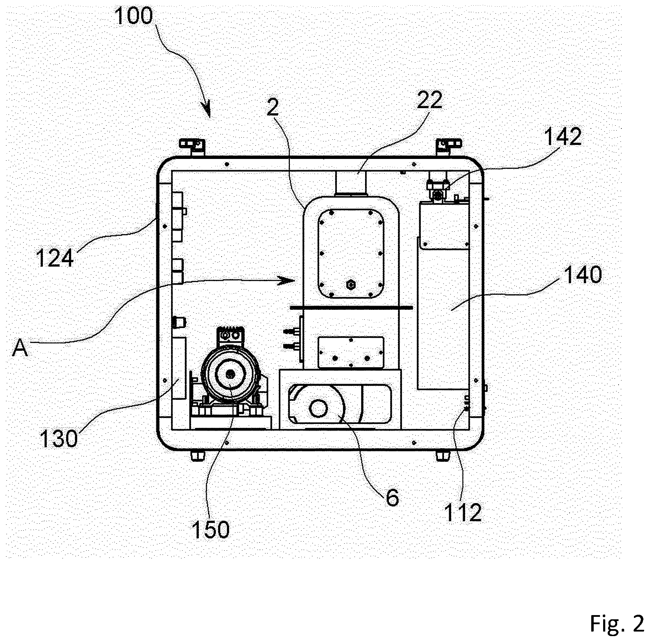

FIG. 1 is a perspective view illustrating a space sterilization apparatus according to the present invention, and FIG. 2 is a front view illustrating the space sterilization apparatus according to the present invention.

As illustrated in FIGS. 1 and 2, the space sterilization apparatus 100 includes an enclosure 110 including an accommodating space formed therein, and an openable cover; a discharge hole 122 formed in an upper portion of the enclosure 110; a storage tank 140 mounted inside the enclosure 110 and storing hydrogen peroxide and supplying the hydrogen peroxide to a hydrogen peroxide vapor generation device; a compressor 150 mounted in a lower inner portion of the enclosure 110 and supplying compressed air to a nozzle; and the hydrogen peroxide vapor generation device (A) that evaporates the hydrogen peroxide supplied from the storage tank 140.

The enclosure 110 has the accommodating space formed therein, and the openable cover on both sides. A main board and a control board 116 are mounted on an upper portion of the enclosure 110 and connected to a control panel 120 to distribute, transmit, and store various data.

The enclosure 110 includes a control panel 120 including a start button 124 and a process indicating lamp formed on a front surface of the enclosure 110, a perforated portion with air permeability formed in a lower front portion, and a filter portion 130 mounted behind the front surface in communication with the perforated portion.

The control panel 120 includes various buttons and also includes an indicating unit for indicating process steps. Preferably, the process indicating unit is formed of an LED (RGB LED).

A plurality of scrubber connectors 112 are formed in a rear portion of the enclosure 110.

The filter unit 130 includes a pre-filter in close contact with the perforated portion of the enclosure 110 and a HEPA filter overlapping with the pre-filter.

The compressor 150 supplies compressed air to a nozzle unit 8 at a high pressure.

A discharge valve 142 for discharging a residue is formed on one upper side of the storage tank 140. The discharge valve 142 is opened and closed according to an operation signal of the control panel 120, and the discharge valve 142 may be opened to discharge the residual hydrogen peroxide and air.

Hydrogen peroxide is used at a concentration of 35%, but other concentrations of hydrogen peroxide may also be used, and embodiments are not limited thereto.

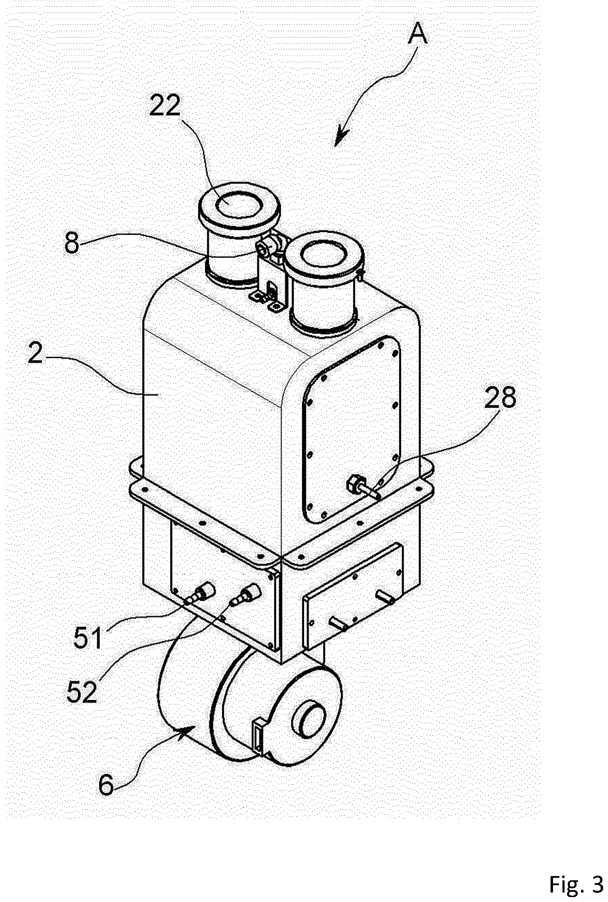

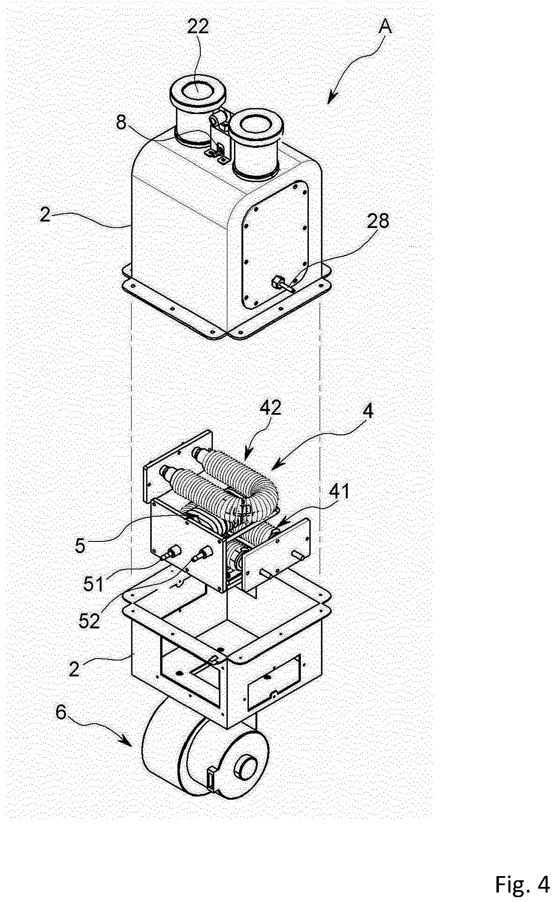

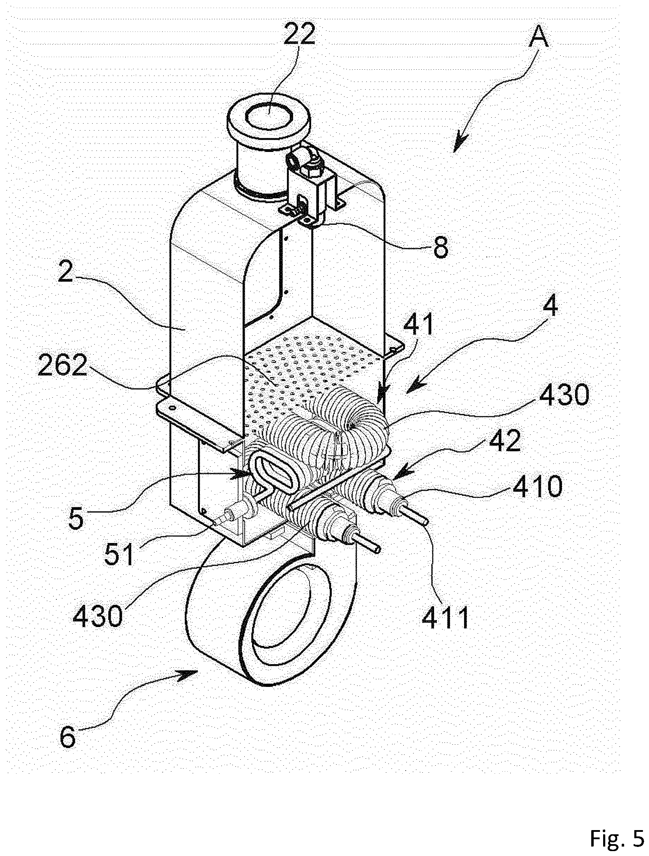

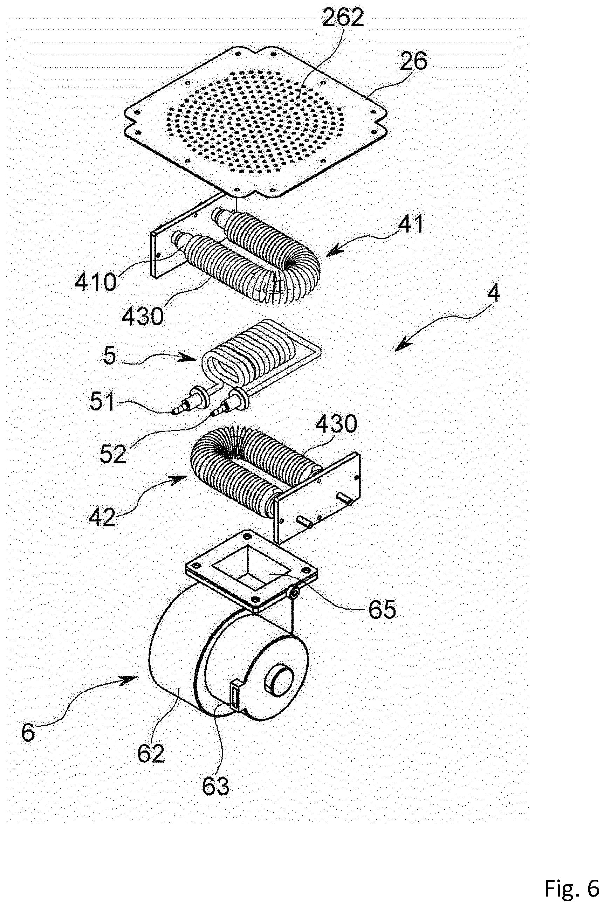

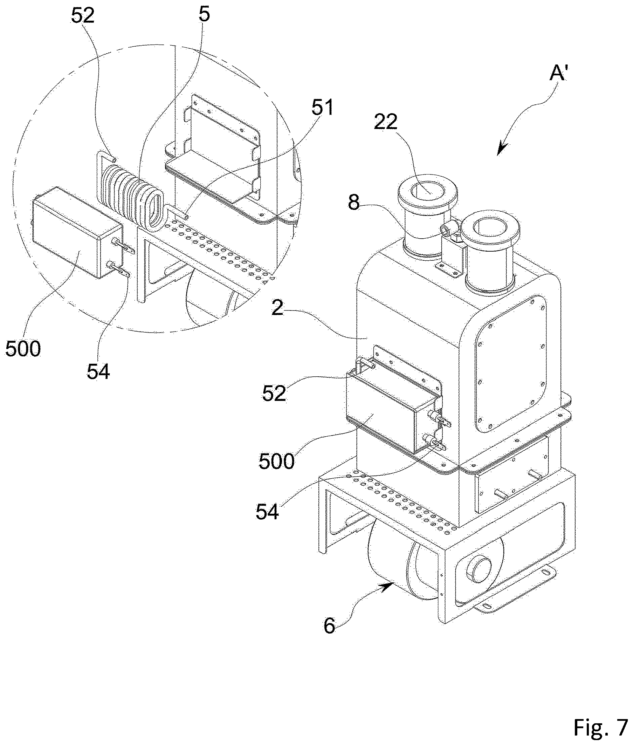

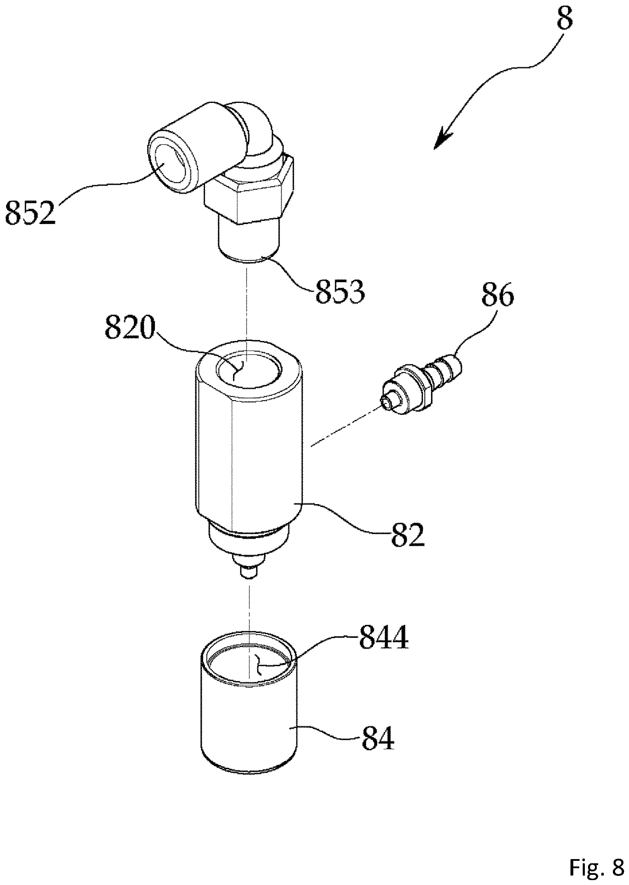

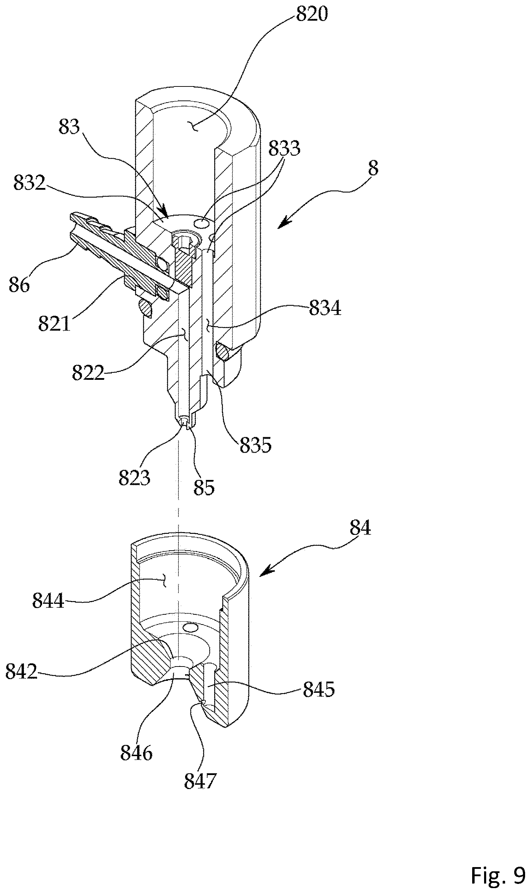

FIG. 3 is a perspective view illustrating the 'hydrogen peroxide vapor generation device' in the space sterilization apparatus according to the present invention, FIG. 4 is an exploded perspective view illustrating the 'hydrogen peroxide vapor generation device' in the space sterilization apparatus according to the present invention, FIG. 5 is a cross-sectional perspective view of the 'hydrogen peroxide vapor generation device' in the space sterilization apparatus according to the present invention, FIG. 6 is an exploded perspective view of a part of the 'hydrogen peroxide vapor generation device' in the space sterilization apparatus according to the present invention, FIG. 7 is an exploded perspective view of the 'nozzle unit' in the space sterilization apparatus according to the present invention, FIG. 8 is a cross-sectional perspective view of the 'nozzle unit' in the space sterilization apparatus according to the present invention, and FIG. 9 is a bottom perspective view of the 'nozzle unit' in the space sterilization apparatus according to the present invention.

As illustrated in FIGS. 3 to 5, the hydrogen peroxide vapor generation device (A) according to the present invention includes an evaporation chamber 2 having a space formed therein and including the nozzle unit 8 and a discharge port 22 which are formed in an upper portion thereof; an evaporation unit 4 formed in a lower inner portion of the evaporation chamber 2 and equipped with a plurality of heating units 41 and 42 and a hydrogen peroxide transfer pipe 5 arranged in a stack; and an air supply unit 6 formed in a lower portion of the evaporation chamber 2 and through which outside air is suctioned, discharged, and fed to the evaporation unit 4.

The evaporation chamber 2 is a hexahedral enclosure, and includes an air flow path through which air is introduced from an opening in the lower portion, rises while being heated, and is discharged through the discharge port 22 in the upper portion.

A hot air plate 26 is formed in the middle portion of the evaporation chamber 2, and at a position corresponding to the upper portion of the evaporation unit 4. The hot air plate 26 is formed in a plate-like shape and has a plurality of through holes 262 formed on the plate, such that the heated air, which passes through the evaporation unit 4 and then rises, is widely distributed as it passes through the plurality of through holes 262 of the hot air plate 26 and uniformly supplied throughout the evaporation chamber 2 and stays therein. The hot air plate 26 is heated to a high temperature by hot air, so that the hydrogen peroxide particles that have not not been split into small particles in the evaporation chamber 2 are split into small particles upon coming into contact with the hot air plate 26.

The evaporation chamber 2 includes a temperature sensor 28 for measuring the internal temperature of the evaporation chamber 2. The temperature sensor 28 checks the temperature to check whether or not the internal temperature of the evaporation chamber 2 is equal to or higher than a set temperature such that, when the temperature is equal to or higher than the set temperature, the spraying step, which is the next step of the preheating step, is executed, and the temperature sensor 28 checks whether or not the internal temperature of the evaporation chamber 2 is equal to or lower than a set temperature such that, when the temperature is equal to or lower than the set temperature, cooling is performed and the operation is terminated.

The plurality of heating units 41 and 42 formed in the lower inner portion of the evaporation chamber 2 include a tube 410 through which a high-temperature fluid flows, and a plurality of heat dissipation fins 430 formed on an outer surface of the tube 410.

The tube 410 may be provided with a heating wire 411 embedded therein or supplied with a high-temperature gas to generate heat, and the heat may be dissipated by the plurality of heat dissipation fins 430 formed on the outer surface of the tube 410.

The tube 410 may have various shapes such as a 'U' shape, for example, and heat dissipation fins 430 are densely formed in both straight and curved portions.

The heat dissipation fins 430 are thin metal plates with high thermal conductivity, and after the plurality of heat dissipation fins 430 are arranged in parallel, the tube is coupled through the heat dissipation fins 430.

The plurality of heating units 41 and 42 include a first heating unit 41 and a second heating unit 42, and the hydrogen peroxide transfer pipe 5 is arranged between the second heating unit 42 and the first heating unit 41 in stack.

Since the hydrogen peroxide transfer pipe 5 is positioned between the plurality of heating units 41 and 42, the hydrogen peroxide transferred through the hydrogen peroxide transfer pipe 5 is also heated to a high temperature during heating of the supplied air.

Meanwhile, FIG. 7 illustrates a hydrogen peroxide vapor generation device (A') according to another embodiment, which may include a heating body 500 formed on an outer side of the evaporation chamber 2 and formed with a heating wire, and the hydrogen peroxide transfer pipe 5 which is mounted inside the heating body 500 and through which hydrogen peroxide is transferred.

The heating body 500 is a cast box member, and includes a heating wire embedded therein, a wire connection terminal 54 formed on the outer side, and the hydrogen peroxide transfer pipe 5 embedded therein.

An inlet port 51 and an outlet port 52 of the hydrogen peroxide transfer pipe 5 are respectively formed outside the heating body 500, and the outlet port 52 is connected to the nozzle unit 8 through a pipe (not illustrated) connected thereto. Accordingly, as the heating body 500 is heated by heating the heating wire, the hydrogen peroxide in the hydrogen peroxide transfer pipe 5 is evaporated and split into small particles. The heating body 500 together with the heating coil may be formed to be wrapped with a casting, and preferably wrapped with a heat dissipating material to prevent heat from being transferred outside. As an example of the heat dissipation material, mica may be used.

The air supply unit 6 includes, formed on one side, an exhaust port 65 coupled in communication with a lower opening of the evaporation chamber 2, and, formed on the other side, a fan housing 62 having an intake port 63 and a BLDC fan rotatably mounted inside the fan housing 62.

The air supply unit 6 feeds the outside air, that is, the carrier gas to the inside of the hydrogen peroxide vapor generation device and the evaporation chamber 2.

The hydrogen peroxide transfer pipe 5 is a pipe through which hydrogen peroxide is transferred, and includes the inlet port 51 and the outlet port 52 formed at both ends, and is spirally formed to increase the circulation time so as to increase the evaporation efficiency of heating by heat exchange. A pipe (not illustrated) connected to the outlet port 52 is connected to the nozzle unit 8 to supply hydrogen peroxide.

Meanwhile, the nozzle unit 8 is mounted in the upper portion of the evaporation chamber 2 and is mounted close to the discharge port 22.

The nozzle unit 8 sprays hydrogen peroxide in a droplet state.

The nozzle unit 8 includes a nozzle body 82, a nozzle holder 84, and a liquid supply nipple 86, and a pneumatic injection unit (not illustrated) is connected to the nozzle body 82 to inject air.

In addition, the hydrogen peroxide gas supplied from the outlet port 52 of the hydrogen peroxide transfer pipe 5 of the hydrogen peroxide vapor generation device A is supplied to the nozzle unit 8 and then sprayed into the evaporation chamber 2.

The nozzle body 82 has both ends open, and includes a passage 820 formed inside, a liquid supply hole 821 which is formed on one side and through which a hydrogen peroxide solution is introduced, and an air distribution unit 83 which distributes and discharges air in the passage 820 formed inside.

The nozzle holder 84 is coupled to the nozzle body 82 and includes a nozzle fixing part 842 to which a nozzle 85 is fitted and coupled.

The liquid supply nipple 86 is coupled to the liquid supply hole 821, and a hose connected to an external hydrogen peroxide solution storage container (not illustrated) is coupled to the liquid supply nipple 86.

The nozzle body 82 includes a liquid discharge path 822 formed therein in communication with the liquid supply hole 821, and a nozzle 85 formed on and protruding from the lower end of the nozzle body 82, in which the nozzle 85 has a discharge hole 823 formed at an end thereof.

The nozzle body 82 includes a nozzle air supply channel 852 formed in an upper opening, and a nozzle air flow channel 853 connected in communication with the nozzle air supply channel 852 and in communication with the nozzle body 82 such that air compressed in the compressor 150 is introduced through the nozzle air supply channel 852 and the nozzle air flow channel 853.

The air distribution unit 83 of the nozzle body 82 includes a round jaw 832 formed on an inner circumferential surface of the passage, air holes 833 formed radially in the round jaw 832, and an air transfer path 834 which is formed inside the nozzle body 82 in communication with the air holes 833 and through which air is transferred, in which an opening 835 at the end of the air transfer path 834 is formed around the nozzle 85.

The opening 835 of the air transfer path 834 of the air distribution unit 83 is formed at a higher position than the discharge hole 823 of the nozzle 85, and the openings 835 of a plurality of air transfer paths 834 are formed on an outer periphery of the nozzle 85 in the circumferential direction.

That is, the plurality of air transfer paths 834 are arranged around the nozzle 85 in the circumferential direction. In an embodiment of the present invention, the openings 835 of the air transfer paths 834 are formed in only a half of the circumference. Of course, it should be noted that the invention is not necessarily limited thereto. Accordingly, the air simultaneously discharged through the plurality of air transfer paths 834 may be sprayed to the outer periphery of the nozzle 85 by an equal spray amount.

The nozzle holder 84 includes an opening formed at an upper portion thereof and coupled to the nozzle body 82, and an air chamber 844 formed therein to be filled with air fed from the air transfer paths 834.

The nozzle fixing part 842 includes the nozzle fixing part 842 which is formed in a center of the bottom of the air chamber 844 and to which a tip of the nozzle 85 is coupled. In addition, a plurality of air slots 845 are formed around the nozzle fixing part 842, so that air can be transferred through the plurality of air slots 845.

An air discharge hole 847 is formed at a distal end of the air slot 845 in communication with the same, and there are a plurality of air discharge holes 847 formed in the circumferential direction and inclined with respect to the nozzle fixing part 842.

It is preferable that the air jet through the plurality of air slots 845 is directed to be jetted toward the nozzle fixing part 842.

In addition, a droplet generating unit 846 is formed in communication with the nozzle fixing part 842 and having a flare shape that increases in diameter toward the outside of the nozzle fixing part 842.

The droplet generating unit 846 is in the flare shape and thus expands from the top to the bottom, with the air discharge hole 847 formed on the inclined surface of the droplet generating unit 846. Therefore, the hydrogen peroxide particles are more finely split by the high-pressure air discharged through the air discharge hole 847, such that hydrogen peroxide droplets are formed.

The process of operating the space sterilization apparatus including the hydrogen peroxide vapor generation device of the present invention will be described.

The hydrogen peroxide supplied by a metering pump is vaporized in the process of being transferred to the hydrogen peroxide transfer pipe 5 as it passes through the heating units 41 and 42 or the heating body 500 and then supplied to the nozzle unit 8. That is, the air is heated while passing between the heat dissipation fins 430 of the second heating unit 42, and the heated air is heated to a high temperature at which the hydrogen peroxide particles in the hydrogen peroxide transfer pipe 5 can be split and evaporated. Alternatively, the heating body 500 is heated by the heated heating wire of the heating body 500, so that the hydrogen peroxide in the hydrogen peroxide transfer pipe 5 mounted inside the heating body 500 is evaporated and split into small particles.

Meanwhile, the compressor 150 supplies compressed air to the nozzle unit 8 at high pressure, and the high-pressure compressed air is introduced into the air holes 833 radially formed in the air distribution unit 83 of the nozzle body 82, transferred to the air transfer path 834, and discharged through the opening 835. The nozzle holder 84 is coupled to the nozzle body 82, and the high-pressure air discharged through the opening 835 is filled in the air chamber 844 of the nozzle holder 84, and the high-pressure air is ejected toward the nozzle fixing part 842 through the air discharge hole 847 formed at the end of the air slot 845, and the high-pressure compressed air is ejected by an equal spray amount to the outer periphery of the nozzle 85, whereby the hydrogen peroxide particles sprayed from the nozzle 85 are split into smaller pieces.

Meanwhile, air is introduced into the evaporation chamber 2 by the air supply unit 6. The introduced air passes through the evaporation unit 4.

Then, the air is re-heated as it passes through the first heating unit 41, rises, and passes through the plurality of through holes 262 of the hot air plate 26 to be widely distributed and uniformly supplied into the evaporation chamber 2.

The air in the evaporation chamber 2 comes into contact with the hydrogen peroxide gas sprayed from the nozzle unit 8. The hydrogen peroxide in the form of droplets sprayed from the nozzle 85 comes into contact with high-temperature air, and the high-temperature air surrounds the hydrogen peroxide particles to form an air cushion. The hydrogen peroxide vapor forming the air cushion is discharged to the outside through the discharge port 22 of the evaporation chamber.

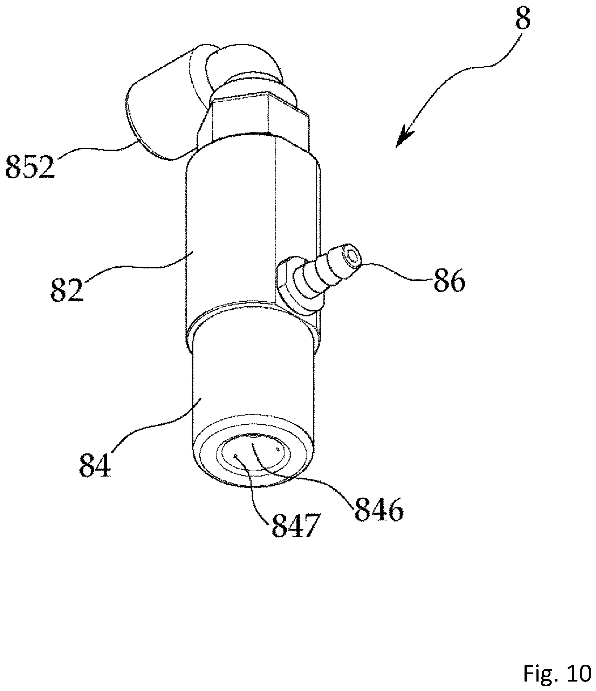

FIG. 10 is a process flow diagram illustrating a sterilization method using the hydrogen peroxide vapor generation device according to the present invention.

The method starts as the start button is operated, and is performed in the order of preheating step, spraying step, sterilizing step, and cooling and terminating step.

### [Preheating step (S1)]

A BLDC fan of the air supply unit 6 is turned on and driven.

Heaters of the second heating unit 42 and the first heating unit 41 are turned on and operated.

The temperature sensor 28 checks the temperature to determine whether the internal temperature of the engine is equal to or higher than a set temperature, and when it is equal to or higher than the set temperature, the next step, that is, the spraying step is executed. Preferably, the temperature is equal to or higher than 70°C.

### [Spraying step (S2)]

The spraying step includes the same process as the process of operating the space sterilization apparatus including the hydrogen peroxide vapor generation device of the present invention.

First, the compressor 150 is turned on and operated.

The discharge valve 142 is turned on and operated to discharge the residue. The discharge valve 142 may be a solenoid valve, but is not limited thereto.

The hydrogen peroxide metering pump is turned and operated, and the discharge valve 142 is turned off. The on-off operation of the discharge valve 142 may be controlled according to a set time. The hydrogen peroxide metering pump is turned on and operated, and hydrogen peroxide is supplied to the hydrogen peroxide transfer pipe 5 in a fixed amount.

The metering pump (not illustrated) and the compressor 150 are turned off.

### [Sterilizing step (S3)]

There is a set time of standby period for space sterilization.

Then the heaters of the second heating unit 42 and the first heating unit 41 are turned off.

Then the scrubber is turned on and operated. The scrubber is turned on and operated for a set time.

### [Cooling and Terminating step (S4)]

The temperature sensor 28 checks whether or not the internal temperature of the evaporation chamber 2 is equal to or lower than a set temperature, and when the temperature is equal to or lower than the set temperature, cooling is performed and the operation is terminated. The set temperature is preferably 40°C or lower.

Then the BLDC fan of the air supply unit 6 is turned off, and the scrubber is turned off.

The present disclosure is not limited by the embodiments described above and the accompanying drawings, and various modifications and applications not illustrated within the scope not departing from the technical spirit of the present disclosure are possible, as well as substitution of components and change to other equivalent embodiments are possible, and accordingly, contents related to modifications and applications of the features of the present disclosure should be construed to be included in a scope of the present disclosure.

### Industrial Applicability

The present invention can be used to sterilize an object to be sterilized.

## Claims

1. A hydrogen peroxide vapor generation device comprising:
an evaporation chamber which has a space formed therein, and includes a nozzle unit and a discharge port which are formed in an upper portion thereof;
an evaporation unit which is formed in a lower inner portion of the evaporation chamber and equipped with a plurality of heating units and a hydrogen peroxide transfer pipe which are arranged in stack; and
an air supply unit which is formed in a lower portion of the evaporation chamber and through which outside air is suctioned, discharged, and fed to the evaporation unit.

2. The hydrogen peroxide vapor generation device according to claim 1, further comprising a heating body formed on an outer side of the evaporation chamber and formed with a heating wire, wherein the hydrogen peroxide transfer pipe is mounted inside the heating body.

3. The hydrogen peroxide vapor generation device according to claim 1, wherein the plurality of heating units includes a first heating unit and a second heating unit, and
the hydrogen peroxide transfer pipe is arranged between the second heating unit and the first heating unit in stack.

4. The hydrogen peroxide vapor generation device according to claim 1, wherein the plurality of heating units includes a tube through which a high-temperature fluid flows, and a plurality of heat dissipation fins formed on an outer surface of the tube.

5. The hydrogen peroxide vapor generation device according to claim 1, wherein the air supply unit includes:
a fan housing having an exhaust port formed on one side, and an intake port formed on the other side, wherein the exhaust port is coupled in communication with a lower opening of the evaporation chamber; and
a fan rotatably mounted inside the fan housing.

6. The hydrogen peroxide vapor generation device according to claim 1, wherein the evaporation chamber includes a hot air plate formed at a position corresponding to an upper portion of the evaporation unit.

7. The hydrogen peroxide vapor generation device according to claim 1, wherein the nozzle unit includes:
a nozzle body having both ends open, and including a passage formed inside, a liquid supply hole formed on one side, through which a hydrogen peroxide solution is introduced, and an air distribution unit distributing and discharging air in the passage formed inside;
a nozzle holder coupled to the nozzle body and including a nozzle fixing part to which the nozzle is fitted and coupled; and
a liquid supply nipple coupled to the liquid supply hole and coupled to a hose connected to an external hydrogen peroxide solution storage container.

8. The hydrogen peroxide vapor generation device according to claim 7, wherein the nozzle body includes a liquid discharge path formed therein in communication with the liquid supply hole, and a nozzle formed on and protruding from a lower end of the nozzle body, wherein the nozzle has a discharge hole formed at an end thereof.

9. The hydrogen peroxide vapor generation device according to claim 7, wherein the air distribution unit includes
a round jaw formed on an inner circumferential surface of a passage, air holes radially formed in the round jaw, and
an air transfer path which is formed inside the nozzle body in communication with the air holes and through which air is transferred, wherein an opening at the end of the air transfer path is formed around the nozzle.

10. The hydrogen peroxide vapor generation device according to claim 9, wherein opening of the air transfer path of the air distribution unit is formed at a higher position than the discharge hole of the nozzle, and openings of a plurality of air transfer paths are formed on an outer periphery of the nozzle in a circumferential direction.

11. The hydrogen peroxide vapor generation device according to claim 7, wherein the nozzle holder includes an opening formed at an upper portion thereof and coupled to the nozzle body, and an air chamber formed therein to be filled with air fed from the air transfer paths, and
the nozzle fixing part includes
a nozzle fitting hole which is formed in a center of a bottom of the air chamber and into which a tip of the nozzle is coupled,
a plurality of air slots which are formed around the nozzle fitting hole and through which air is transferred, and
a plurality of air discharge holes formed in communication with ends of the air slots and inclined with respect to the nozzle fitting hole.

12. The hydrogen peroxide vapor generation device according to claim 11, comprising a droplet generating unit formed in communication with the nozzle fixing part and having a flare shape that increases in diameter toward the outside of the nozzle fixing part, wherein the air discharge holes are formed in an inclined surface of the droplet generating unit.

13. A space sterilization apparatus comprising:
a hydrogen peroxide vapor generation device according to any one of claims 1 to 12;
an enclosure including an accommodating space formed therein, and an openable cover;
a discharge hole formed in an upper portion of the enclosure;
a hydrogen peroxide storage tank for supplying hydrogen peroxide to the hydrogen peroxide vapor generation device; and
a compressor for pressurizing and discharging hydrogen peroxide.

14. A space sterilization apparatus comprising:
an enclosure including an accommodating space formed therein, and an openable cover;
a discharge hole formed in an upper portion of the enclosure;
a hydrogen peroxide vapor generation device formed inside the enclosure;
a hydrogen peroxide storage tank for supplying hydrogen peroxide to the hydrogen peroxide vapor generation device; and
a compressor for pressurizing and discharging hydrogen peroxide,
wherein the hydrogen peroxide vapor generation device includes:
an evaporation chamber which has a space formed therein, and includes a nozzle unit and a discharge port which are formed in an upper portion thereof;
an evaporation unit formed in a lower inner portion of the evaporation chamber and equipped with a plurality of heating units arranged in a stack, a heating body formed on an outer side of the evaporation chamber and formed with a heating wire, and a hydrogen peroxide transfer pipe which is mounted inside the heating body and through which hydrogen peroxide is transferred; and
an air supply unit which is formed in a lower portion of the evaporation chamber and through which outside air is suctioned, discharged, and fed to the heating units.

15. A space sterilization apparatus comprising:
an enclosure including an accommodating space formed therein, and an openable cover;
a discharge hole formed in an upper portion of the enclosure;
a perforated portion formed in the enclosure and formed with air permeability, and a filter unit mounted in communication with the perforated portion;
a hydrogen peroxide vapor generation device formed inside the enclosure;
a hydrogen peroxide storage tank for supplying hydrogen peroxide to the hydrogen peroxide vapor generation device; and
a compressor for pressurizing and discharging hydrogen peroxide,
wherein the hydrogen peroxide vapor generation device includes:
an evaporation chamber which has a space formed therein, and includes a nozzle unit and a discharge port which are formed in an upper portion thereof;
an evaporation unit formed in a lower inner portion of the evaporation chamber and including a plurality of heating units arranged in a stack, and a hydrogen peroxide transfer pipe which is arranged between the plurality of heating units in stack and through which hydrogen peroxide is transferred; and
an air supply unit which is formed in a lower portion of the evaporation chamber and through which outside air is suctioned, discharged, and fed to the heating units.

16. The space sterilization apparatus according to claim 14 or 15, wherein the air supply unit includes:
a fan housing having an exhaust port formed on one side, and an intake port formed on the other side, wherein the exhaust port is coupled in communication with a lower opening of the evaporation chamber; and
a fan rotatably mounted inside the fan housing.

17. The space sterilization apparatus according to claim 14 or 15, wherein the evaporation chamber includes a hot air plate formed at a position corresponding to an upper portion of the evaporation unit.

18. The space sterilization apparatus according to claim 14 or 15, wherein the nozzle unit includes:
a nozzle body having both ends open, and including a passage formed inside, a liquid supply hole formed on one side, through which a hydrogen peroxide solution is introduced, and an air distribution unit distributing and discharging air in the passage formed inside;
a nozzle holder coupled to the nozzle body and including a nozzle fixing part to which the nozzle is fitted and coupled; and
a liquid supply nipple coupled to the liquid supply hole and coupled to a hose connected to an external hydrogen peroxide solution storage container.

19. The space sterilization apparatus according to claim 18, wherein the nozzle body includes a liquid discharge path formed therein in communication with the liquid supply hole, and a nozzle formed on and protruding from a lower end of the nozzle body, wherein the nozzle has a discharge hole formed at an end thereof.

20. The space sterilization apparatus according to claim 18, wherein the air distribution unit includes
a round jaw formed on an inner circumferential surface of a passage, air holes radially formed in the round jaw, and
an air transfer path which is formed inside the nozzle body in communication with the air holes and through which air is transferred, wherein an opening at the end of the air transfer path is formed around the nozzle.

21. A method of sterilizing using the space sterilization apparatus according to claim 13, the method comprising:
a preheating step in which the fan of the air supply unit is turned on and driven, the second heating unit and the first heating unit are turned on and operated, and the temperature sensor checks a temperature;
an spraying step in which the compressor is turned on, the discharge valve is turned on to discharge a residue, the metering pump is turned on, the discharge valve is turned off, and the metering pump and the compressor are turned off;
a sterilizing step in which the heater is turned off, and the scrubber is turned on; and
a cooling and terminating step in which the internal temperature of the evaporation chamber is checked, the fan of the air supply unit is turned off, and the scrubber is turned off.

22. A method of sterilizing using the space sterilization apparatus according to claim 14 or 15, the method comprising:
a preheating step in which a fan of the air supply unit is turned on and driven, a second heating unit and a first heating unit are turned on and operated, and a temperature sensor checks a temperature;
an spraying step in which the compressor is turned on, a discharge valve is turned on to discharge a residue, a metering pump is turned on, the discharge valve is turned off, and the metering pump and the compressor are turned off;
a sterilizing step in which a heater is turned off, and a scrubber is turned on; and
a cooling and terminating step in which the internal temperature of the evaporation chamber is checked, the fan of the air supply unit is turned off, and the scrubber is turned off.

23. The method of sterilizing using the space sterilization apparatus according to claim 22, wherein the preheating step includes determining whether or not the internal temperature of the evaporation chamber is 70°C or higher, and
the cooling and terminating step includes checking, with the temperature sensor, to determine whether or not the internal temperature of the evaporation chamber is 40°C or lower.
